# EUROPEAN PATENT SPECIFICATION

(11) **EP 3 655 012 B1**
(45) Date of publication and mention of the grant of the patent: **05.07.2023**
(21) Application number: 18749087.5
(22) Date of filing: 23.07.2018
(51) Int. Cl.: A61K 38/13, C07K 7/64, A61P 13/12, A61P 29/00, A61P 41/00, A61P 43/00

(54) **CYCLOSPORIN ANALOGUES AND USES THEREOF**
CYCLOSPORINANALOGA UND VERWENDUNGEN DAVON
ANALOGUES DE LA CICLOSPORINE ET LEURS UTILISATIONS

(30) Priority: 21.07.2017 GB 201711749
(43) Date of publication of application: 27.05.2020
(73) Proprietor: Cypralis Limited, Cambridge CB4 0WS (GB)
(72) Inventor: PEEL, Michael, Cambridge CB4 0WS (GB); ZENG, Li, Shanghai 200037 (CN); YU, Shengqiang, Shanghai 200003 (CN)
(74) Representative: Stratagem IPM Limited
(86) International application number: PCT/GB2018/052066
(87) International publication number: WO 2019/016572

(56) References cited:
- WO-A1-99/65933
- WO-A1-2008/143996
- WO-A2-2006/039668
- WO-A2-2007/136759
- CN-A- 106 902 346
- CN-A- 106 902 347

## Description

### Field of the invention

The present invention relates to cyclosporin analogues for use in and the treatment or prevention of diseases or disorders. In particular, the invention relates to the use of a cyclosporin analogue of Formula 1, in the treatment or prevention of acute or chronic inflammatory disorders, including acute kidney injury, chronic or acute pancreatitis and ischaemia-reperfusion injury.

Acute inflammation is well recognized to involve the complex interaction of various cellular (neutrophils, macrophages) and extracellular (complement, histamine) factors that act in response to PAMP (pathogen-activated molecular patterns) and DAMP (damage-activated molecular patterns) signals to resolve the originating insult. Cyclophilin A has been demonstrated to function as a chemokine to facilitate leukocyte migration in support of an inflammatory response and blockade of cyclophilin A was shown to be beneficial in animal models of acute inflammation. More recently a severe form of inflammation that is accompanied by cell death and tissue necrosis has been described. A significant body of evidence now supports the opening of a pore at the mitochondrial membrane, termed the Mitochondrial Permeability Transition Pore (MPTP), as being critical to the onset and maintenance of this necrotic inflammation. A key regulator of this MPTP opening is Cyclophilin D (CypD), and inhibitors of CypD have shown good activity in preventing tissue damage associated with necrotic inflammation. Opening of the MPTP, and subsequent initiation of necrotic cell death, is triggered by elevated intracellular calcium levels that result from a variety of factors including oxidative stress, hypoxia, bile salt toxins, etc. Notably, genetic ablation, or pharmacological inhibition, of CypD was found to be protective toward tissue degradation due to ischemia-reperfusion injury of cardiac tissue suggesting that CypD inhibition is a viable drug target for ischemia-reperfusion injury more generally.

Renal ischemia results from arterial occlusion, shock and kidney transplantation and can lead to renal cell death and kidney failure. A further source of tissue damage associated with ischemia occurs during the process of organ transplantation. Following removal of the donor organ the tissue is inevitably subject to oxygen starvation as a result of loss of blood flow, and damage to the ischemic tissue ensues upon re-initiation of flow. A compound that is able to prevent tissue damage during the reperfusion process would improve the viability of the transplanted organ. A preferred profile for a compound to be used as a tissue protectant would include potent inhibition of CypD, prevention of MPTP opening following ischemic stress, and solubility sufficient to be added to the preservation solutions typically used during organ transportation, in concentrations high enough to protect the tissue.

In studies carried out using cyclophilin D knockout mice as well as pharmacological strategies with cyclophilin inhibitors it has been unambiguously demonstrated that opening of the mitochondrial permeability transition pore (MPTP), a non-specific channel in the inner mitochondrial membrane, is fundamental to multiple forms of acute pancreatitis and validates the MPTP as a drug target for this disease (Mukherjee R, et al. Gut 2016;65:1333-1346). In-vitro studies have demonstrated that injury to pancreatic acinar cells is the key event in the initiation of necrotic tissue degeneration associated with acute pancreatitis. Specifically, treatment of isolated acinar cells with toxic insults such as bile salts, alcohol or hyperstimulation of a hormone receptor (key triggers for onset of acute pancreatitis) results in a necrotic cell death that is dependent upon activation of the mitochondrial permeability transition pore. Pancreatic acinar cells isolated from mice in which the cyclophilin D gene is deleted were shown to be resistant to these toxic challenges. Translation of these findings to animal models showed that the cyclophilin D knockout animals were protected from the effects of these pancreatic toxins. Studies using a cyclophilin D inhibitor further supported the targeting of cyclophilin D as an approach to a possible treatment for acute pancreatitis.

### Background to the invention

Cyclosporin A is a compound well known for its immunosuppressive properties, but other biological properties have also been described. Cyclosporin A has the following chemical structure:

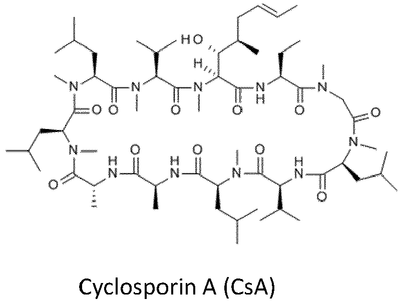

Biologically active derivatives of Cyclosporin A have also been made. For example, US 6,583,265, EP 0 484 281 and EP 0 194 972 describes cyclosporin derivatives having various properties including immunosuppressive, antiparasitic and antiviral properties.

US 6 583 265 describes cyclosporin derivatives with modifications made at position 3 of the cyclosporin macrocycle. In particular, US 6 583 265 discloses Compound 1:

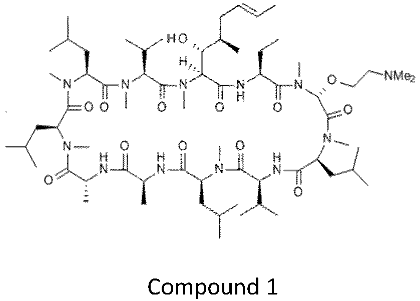

This compound is example 27 in the patent US 6 583 265, which includes many hundreds of named compounds having modifications at various positions around the ring. However no biological testing data or particular uses are described for this compound or related analogues. When the applicants tried to synthesise said compound, using the published route used to prepare Compound 27 in US 6 583 265, the method was not effective. Many attempts were made to replicate the methodology in US 6,583,265 without great success. Without being bound by theory, it is believed that the dimethyl amino group (being basic) reacted preferentially with the acid catalyst. The acid catalyst was thereby prevented from activating the loss of the leaving group, inhibiting the progress of the reaction. There is thus some doubt regarding whether Example 27 was previously synthesised, and therefore doubts as to whether this prior art is actually an enabling disclosure for the preparation of Compound 1.

WO 99/65933 (C-Chem AG) relates to novel cyclosporins compounds of formula (I) and their pharmaceutically acceptable salts wherein the letters A to L have the meaning indicated in the description and processes for their preparation and their uses,

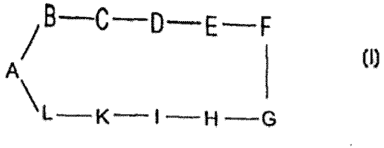

WO 2007/136759 (Scynexis Inc.) relates to a methods for the treatment and prevention of ocular disorders, the methods comprising administering a compound of general Formula (I): 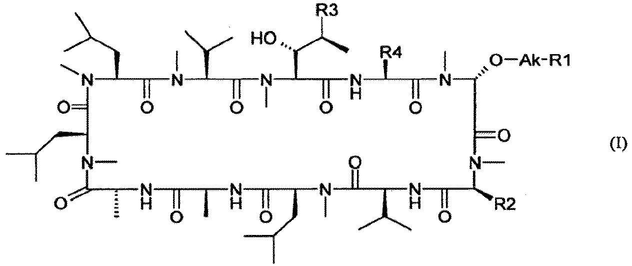 wherein R¹, R², R³, R⁴ and Ak are as defined in the specification.

CN 106 902 347 A (Waterstone Pharmaceuticals Wuhan Co. Ltd.) relates to application of a cyclophilin inhibitor, and particularly provides application of a cyclophilin inhibitor or pharmaceutically acceptable salt or solvate thereof in preparation of medicines. The medicines are used for treating and/or preventing diseases caused by hepatitis B virus infection.

### Statements of invention

Applicants have synthesised compounds previously suggested in the prior art and surprisingly discovered that Compound 1 is a particularly good Cyclophilin D inhibitor. Thus Compound 1 may be used in the treatment of conditions benefiting from inhibitions of Cyclophilin D activity. According to an aspect of the invention, there is provided a compound for use in the treatment or prevention of acute or chronic inflammatory disorders wherein the compound is Compound 1 of formula: 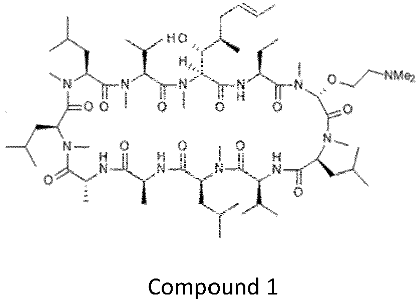
or a salt thereof,
and wherein the disorder is selected from acute kidney injury, chronic or acute pancreatitis and ischaemia-reperfusion injury wherein the ischaemia-reperfusion injury occurs after reattachment of a severed body part or during the process of organ transplantation.

In an embodiment, the acute or chronic inflammatory disorder is for example acute kidney injury.

In an embodiment, the acute or chronic inflammatory disorder is for example chronic pancreatitis.

In an embodiment, the acute or chronic inflammatory disorder is for example acute pancreatitis.

In an embodiment, the acute or chronic inflammatory disorder is for example ischaemia-reperfusion injury.

It has been found that Compound 1 is surprisingly efficacious in the treatment or prevention of both acute kidney injury, ischaemia-reperfusion injury (IRI) and pancreatitis.

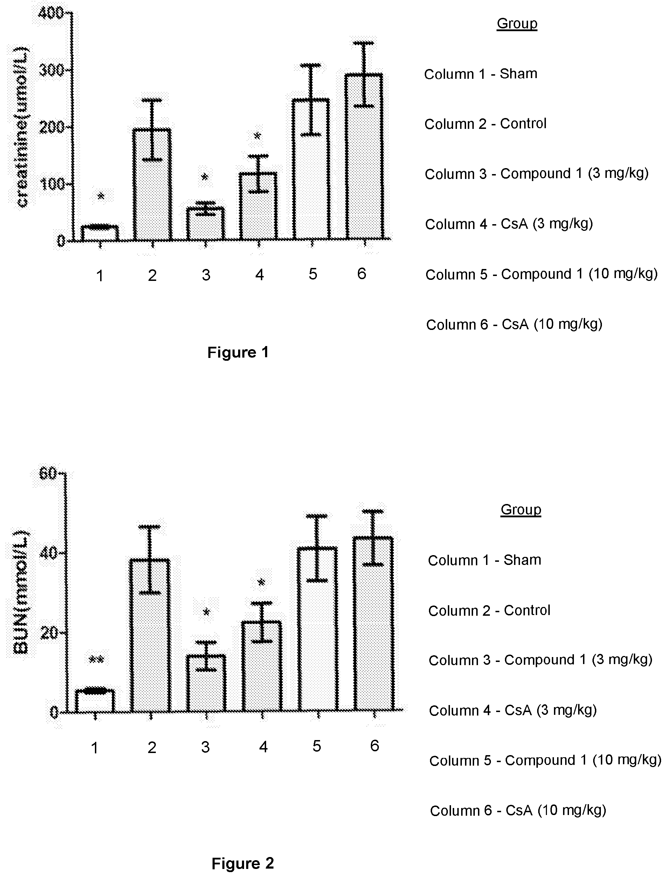

Compound 1 can be used in the treatment or prevention of acute or chronic inflammatory disorders, including acute kidney injury and ischaemia-reperfusion injury. Compound 1 can be used in the treatment or prevention of acute kidney injury. Compound 1 can be used in the treatment of ischaemia-reperfusion injury associated with the re-attachment of severed body parts. The data provided with this application, shown graphically in Figures 1 and 2, shows that Compound 1 significantly outperformed the well-established comparative example Cyclosporin A and other closely related analogues. indeed in the challenging test performed, where blood was deprived from an organ for thirty minutes, Compound 1 gave surprisingly good results, indeed showing a near complete reversal in the expected damaged to the affected organs.

Compound 1 shows a remarkable potency as an inhibitor of Cyclophilin D. As seen in table 1, compound 1 (entry 4) shows an EC₅₀ of 24 nM against cyclophilin D. Replacement of one methyl group on the N atom with H (entry 1 is simply NHMe ve NMe₂) reduces the potency by approximately 100-fold, with an EC₅₀ of >2000 nM. Thus compound 1 is a surprisingly potent inhibitor of cyclophilin D and Mitochondrial Permeability Transition (MPT).

In an embodiment of the compound for use as mentioned herein above, the dose of the compound is 0.1 to 10 mg/kg. In an embodiment of the compound for use as mentioned herein above, the dose of the compound is 1 to 3 mg/kg. In these dose ranges the compound is especially effective.

Salts of the invention may result from the addition of acids to Compound 1. The resultant acid addition salts include those formed with acetic, 2,2-dichloroacetic, citric, lactic, mandelic, glycolic, adipic, alginic, aryl sulfonic acids (e.g., benzenesulfonic, naphthalene-2-sulfonic, naphthalene-1,5-disulfonic and p-toluenesulfonic), ascorbic (e.g. L-ascorbic), L-aspartic, benzoic, 4-acetamidobenzoic, butanoic, (+) camphoric, camphor-sulfonic, (+)-(1S)-camphor-10-sulfonic, capric, caproic, caprylic, cinnamic, citric, cyclamic, dodecylsulfuric, ethane-1,2-disulfonic, ethanesulfonic, 2-hydroxyethanesulfonic, formic, fumaric, galactaric, gentisic, glucoheptonic, gluconic (e.g. D-gluconic), glucuronic (e.g. D-glucuronic), glutamic (e.g. L-glutamic), α-oxoglutaric, glycolic, hippuric, hydrobromic, hydrochloric, hydriodic, isethionic, lactic (e.g. (+)-L-lactic and (±)-DL-lactic), lactobionic, maleic, malic (e.g. (-)-L-malic), (±)-DL-mandelic, metaphosphoric, methanesulfonic, 1-hydroxy-2-naphthoic, nicotinic, nitric, oleic, orotic, oxalic, palmitic, pamoic, phosphoric, propionic, L-pyroglutamic, salicylic, 4-amino-salicylic, sebacic, stearic, succinic, sulfuric, tannic, tartaric (e.g.(+)-L-tartaric), thiocyanic, undecylenic and valeric acids.

In particular acid addition salts include those derived from mineral acids such as hydrochloric, hydrobromic, phosphoric, metaphosphoric, nitric and sulfuric acids; from organic acids, such as tartaric, acetic, citric, malic, lactic, fumaric, benzoic, glycolic, gluconic, succinic, arylsulfonic acids

The compound for use as mentioned herein above may be administered together with one or more further active substances.

Ischemic injury occurs when the blood supply to an area of tissue is cut off. The incidence of ischemic injury is vast: myocardial infarction, stroke, and other thrombotic events and these affect more than 1.3 million individuals each year in the USA alone.

In addition, ischemic injury also occurs during surgery when blood vessels are crossclamped, and in organs for transplantation. The length of time a tissue can survive oxygen deprivation varies, but eventually ischemic tissue becomes necrotic.

Reperfusion (reoxygenation) injury is the tissue damage caused when the blood supply returns to the tissue after a period of ischemia or lack of oxygen (anoxia, hypoxia). Without being bound by theory, it is believed that the absence of oxygen and nutrients from the blood during the ischemic period creates a condition in which the restoration of circulation results in inflammation and oxidative damage.

In organ transplantation, there is a period of time between removing an organ from the donor's blood supply until the reconnection of the organ to the donor recipient's blood supply. During this period there is the potential for ischaemia-reperfusion injury. In some cases, organs may need to be transported long distances to the location of surgery, increasing the likelihood of organ damage.

In accidents involving severed limbs, there is a period of time between the severing of the body part from the blood supply until the reconnection of the body part to the blood supply. During this period there is the potential for ischaemia-reperfusion injury. In some cases, body part and patients may need to be transported long distances to the location of surgery, increasing the likelihood of damage before, during and after re-attachment.

The invention provides for administration of the compound for use mentioned herein above to prevent this damage to body parts and organs. In particular in the period of time between removing a body part or organ from the donor's blood supply to reconnection to the donor recipient's blood supply or in the case of severed body parts, reattachment. The skilled person will be aware of ways in which the compound for use mentioned herein above can be administered to a body part removed from an individual, be they an organ donor or accident victim. For example, the compound for use mentioned herein above could be added to (or included in) a fluid in which the organ is placed; and/or the compound for use mentioned herein above could be added to (or included in) a fluid that is recirculated in and or through the organ/body part.

In an embodiment of the compound for use as mentioned herein above, the compound for use mentioned herein above is administered to the organ after the removal of the organ from the individual and prior to transplantation or re-attachment. Alternatively, or in addition to, the compound for use mentioned herein above is administered to the donor subject prior to removal of the donor organ. For example, the compound for use mentioned herein above may be administered systemically. An injection is one way to administer a systemic dose of the compound for use mentioned herein above. The compound for use mentioned herein above may also be administered to the recipient after organ transplantation or to the accident victim after re-attachment.

A systemic dose of the compound for use mentioned herein above can be administered to the organ donor prior to organ removal. This allows for the organ to receive a protective dose of the compound for use mentioned herein above prior to removal, thereby preserving the organ by protecting the organ from damage during the removal, and up to and during the process of transplantation into the donor recipient. In the case where more than one organ is being removed from a donor, this systemic dose ensures each organ receives a dose of the compound for use mentioned herein above. A systemic dose is also more likely to provide an even dose of the compound for use mentioned herein above to the organ tissue that is to be transplanted. In the case where the donor is legally dead, the dose can be greater than would normally be given to a living subject.

The compound for use mentioned herein above can be administered shortly before surgery, or during surgery. For example, the compound for use mentioned herein above may be administered up to 8, 7, 6, 5, 4, 3, 2 or 1 hours before surgery

In addition, or in the alternative, the organ recipient or accident victim may receive a dose of the compound for use mentioned herein above prior to receiving the organ or undergoing re-attachment surgery, such that their blood supply contains a protective dose of the compound for use mentioned herein above, thereby preserving the transplanted or re-attached body part from damage after surgery.

The body part may be severed from and re-attached to the same individual, or may be given to a second individual as a transplant. There the body part is severed from a subject, the severing may be complete or partial. Partial severing may be for example severing of the blood supply but the body part remaining attached for example via skin, bone or muscle tissue. The compound for use mentioned herein above may administered (i) to a severed body part; and/or (ii) to the subject prior to re-attachment of the body part; and/or (iii) to the subject during or after re-attachment of the body part.

The invention also has application in non-human subjects e.g. cats, dogs, horses and pigs. The invention also has application in transgenic animals (e.g. transgenic pigs), where such animals have organs suitable for human transplantation.

Compound 1 has been shown in this application to be especially effective in the treatment of acute kidney injury and in preventing or treating IRl. Compound 1 has been shown in this application to be especially effective in the treatment of acute kidney injury and both chronic and acute pancreatitis.

The results displayed in Table 1 demonstrate the unexpectedly high Cyclophilin D inhibition and MPT of Compound 1 (entry 4) relative to similar analogues also known in the art (entries 1-3 and 5-7). A 100 fold improvement in MPT was observed relative to three other closely related compounds (entries 1, 5 and 7) and an over 25 fold improvement was observed relative to the next best performing analogue (entry 3). Compound 1 also displayed superior Cyclophilin D inhibition, with at least a 50 fold improvement relative to all other analogues tested.

In an embodiment of the compound for use as mentioned herein above, the organ can be kidney, pancreas, liver, heart, lungs or intestines. In the case of severed body parts, the body parts may be limbs, hands, feet, fingers or toes.

In an embodiment of the compound for use as mentioned herein above, the dose of the compound is 0.1 to 10 mg/kg; and optionally is 1 to 3 mg/kg. In the case of where an organ or body part is bathed in a fluid containing the compound for use mentioned herein above, or this fluid is being recirculated, the concentration of the compound may be higher or lower as required by need.

In an embodiment of the compound for use as mentioned herein above, the compound is formulated in Cremophor/saline/DMSO.

According to one aspect of the disclosure, there is provided a method of preparing Compound 1, the method comprising a product forming reaction, the reaction comprising copper triflate and N,N-dimethylaminoethanol.

Surprisingly, it has been found that Compound 1 is made readily available by the above mentioned method. For example, many failed attempts were made by the present applicant to replicate the methodology disclosed in US 6 583 265, and in the end the approach in US 6 583 265 was abandoned, being unviable.

In an embodiment, the reaction comprises a drying agent and/or is performed under substantially anhydrous conditions. Optionally the drying agent is molecular sieves. Optionally the molecular sieves are 3 A molecular sieves.

In an embodiment, N,N-dimethylaminoethanol reacts with a cyclosporine precursor compound comprising a labile group, wherein the labile group is lost in the reaction. Optionally, the labile group is bonded to the precursor compound by a -S- bond. Further optionally the labile group is a thiopyridyl group or a mercaptobenzthiazole-2-ylthio group.

The scope of the invention is defined by the claims. The references to methods of treatment in the summary and detailed description of the invention in this description are to be interpreted as references to the compounds, pharmaceutical compositions and medicaments of the present invention for use in a method for treatment of the human (or animal) body by therapy.

### Brief description of the drawings

Figure 1 shows the inhibitory and/or protective effect of Compound 1, and of comparative compound CsA, on induced Acute Kidney Injury in rats, by measuring blood serum Creatinine concentrations.
Figure 2 shows the inhibitory and/or protective effect of Compound 1, and of comparative compound CsA, on induced Acute Kidney Injury in rats by measuring Blood Urea Nitrogen (BUN) concentrations.

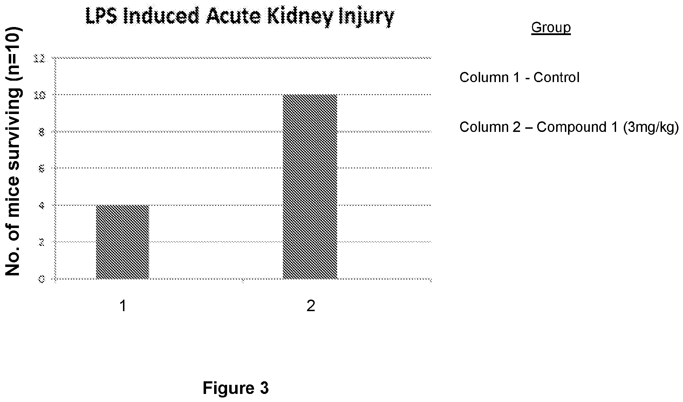

Figure 3 shows the inhibitory and/or protective effect of Compound 1 against LPS induced Acute Kidney injury.

### Detailed Description

The invention will now be illustrated by the following examples.

### Experimental methods and results

The skilled person will recognise that compounds of Formula 1 of formula: 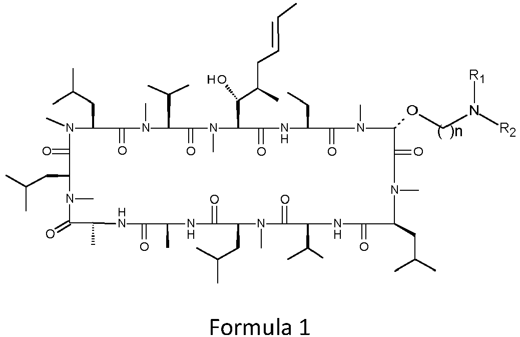
wherein n is 2-5, and
R₁ and R₂ are independently selected from H or C₁-C₄ alkyl, wherein R₁ and R₂ may be joined together to form a C₃-C₅ heteroalkyl ring,
may be prepared in a variety of ways. The route below is merely illustrative of a way that could be employed for the synthesis of Compound 1. That said, the route used to prepare Compound 1 in US 6 583 265 was not effective. Many attempts were made to replicate the methodology in US 6 583 265 without great success. Without being bound by theory, it is believed that the dimethyl amino group (being basic) reacted preferentially with the acid catalyst. The acid catalyst was thereby prevented from activating the loss of the leaving group, inhibiting the progress of the reaction.

Compounds of general Formula **1** can be conveniently prepared using several pathways. In one instance (Scheme 1), reaction of compound **2**, in which R is lower alkyl, with a carbonyl compound and a reducing agent can perform a reductive amination procedure to a give the desired compounds. Preferably the carbonyl compound is a lower alkyl aldehyde or ketone and the reducing agent is a metal borohydride. More preferably the aldehyde is formaldehyde, acetaldehyde or propionaldehyde and the ketone is acetone, 2-butanone and the like. Preferably the reducing agent is sodium triacetoxyborohydride or sodium cyanoborohydride.

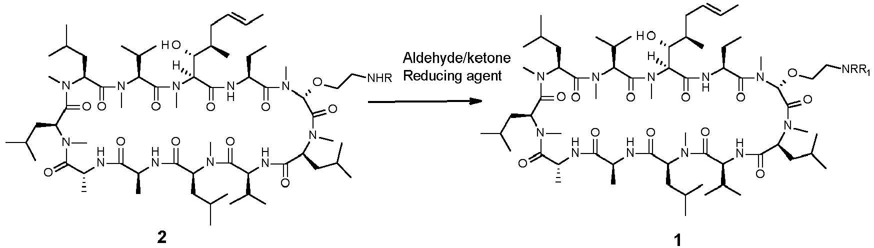

The amine compound **2** can be conveniently prepared from a suitably protected ethanolamine compound such as **3**, wherein R is hydrogen or lower alkyl, by treating said compound with conditions known to remove the protecting group and yield the free amine compound. Suitable protecting groups that can be removed in the presence of other functional groups in the molecule include tert-butoxycarbonyl (BOC), 9-fluorenylmethyloxycarbonyl (FMOC) and the like. Preferably the protecting group is tert-butoxycarbonyl (BOC) and the conditions employed for removal of the BOC group involve treatment with acid, such as trifluoroacetic acid.

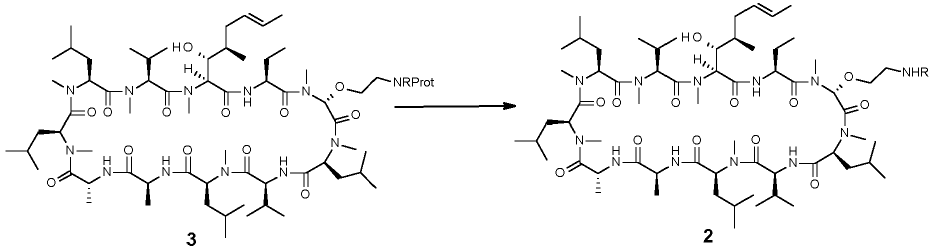

### Step 1: Preparation of [2'-(2-Thiopyridyl)-Sar]3-cyclosporine A

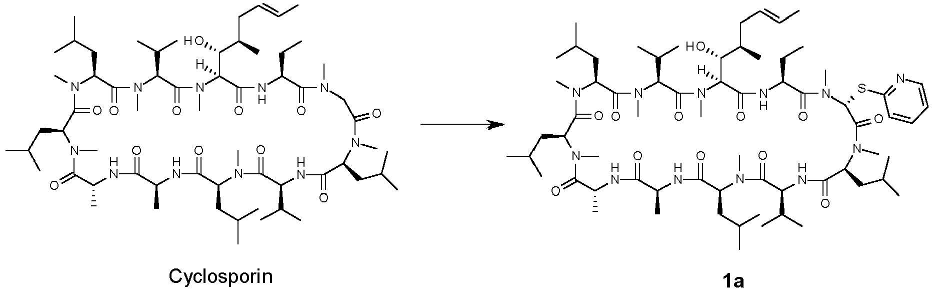

### [2'-(2-Thiopyridyl)-Sar]³-cyclosporine A (1a)

To a dry 1L flask was added cyclosporine A (20 g, 16.6 mmol), anhydrous lithium chloride (21.1 g, 499 mmol) and dry THF (500 mL), the flask was flushed with argon and the mixture was cooled to -45 C. In a separate flask, diisopropylamine (13.5 g, 133 mmol) was dissolved in dry THF (120 mL) and cooled to -78 C. To this flask was added n-butyllithium (53.2 mL of a 2.5 M solution, 133 mmol) and the resulting solution was stirred at -78 C for 20 min. Using a canula, the solution of lithium diisopropylamide was transferred to the solution of cyclosporine and the resulting mixture was stirred at -45 C for 90 min. A solution of 2-pyridyldisulfide (11 g, 49.9 mmol) in dry THF (20 mL) was added dropwise and the resulting mixture was allowed to warm to room temperature overnight. The reaction was quenched by the careful addition of saturated NaCl solution (200 mL) and the resulting organic layer was separated. The aqueous layer was extracted with ethyl acetate (3 × 100 mL) and the combined organic fractions were washed with 3N NaOH (2 × 100 mL), saturated NH₄Cl (100 mL) and saturated NaCl (100 mL) followed by drying over anhydrous Na₂SO₄ and evaporation. The title compound was isolated by silica gel chromatography as a solid, 7.18g. ¹H NMR (400 MHz, CHLOROFORM-d) d 8.45 (ddd, *J*=0.88, 1.73, 4.90 Hz, 1H), 7.98 (d, *J*=9.66 Hz, 1H), 7.65-7.73 (m, 1H), 7.59 (dt, *J*=1.85, 7.71 Hz, 1H), 7.51 (ddd, *J*=0.76, 1.68, 6.44 Hz, 0H), 7.45 (d, *J*=8.54 Hz, 1H), 7.35 (ddd, *J*=1.73, 6.97, 8.77 Hz, 0H), 7.25 (s, 0H), 7.17 (d, *J*=7.96 Hz, 1H), 7.09-7.15 (m, 2H), 6.72 (dt, *J*=1.17, 6.71 Hz, 0H), 5.70 (dd, *J*=4.29, 10.88 Hz, 1H), 5.50 (d, *J*=6.39 Hz, 1H), 5.32-5.38 (m, 1H), 5.28 (dd, *J*=3.88, 11.74 Hz, 1H), 5.13 (d, *J*=10.88 Hz, 1H), 4.97-5.11 (m, 2H), 4.84 (dq, *J*=7.03, 7.24 Hz, 1H), 4.69 (t, *J*=9.15 Hz, 1H), 4.54 (quin, *J*=7.31 Hz, 1H), 4.13 (q, *J*=7.16 Hz, 0H), 3.81 (dt, *J*=1.00, 5.75 Hz, 1H), 3.59-3.72 (m, 1H), 3.50 (s, 2H), 3.38 (s, 2H), 3.26 (s, 2H), 3.13 (s, 5H), 2.70 (d, *J*=1.07 Hz, 5H), 2.34-2.54 (m, 1H), 1.92-2.23 (m, 4H), 1.55-1.85 (m, 11H), 1.19-1.54 (m, 11H), 1.12 (d, *J*=6.54 Hz, 2H), 0.78-1.07 (m, 30H), 0.73 (d, 3H).

### Step 2: Preparation of [2'-(2-Dimethylaminoethoxy)-Sar]3-cyclosporine A (Compound 1)

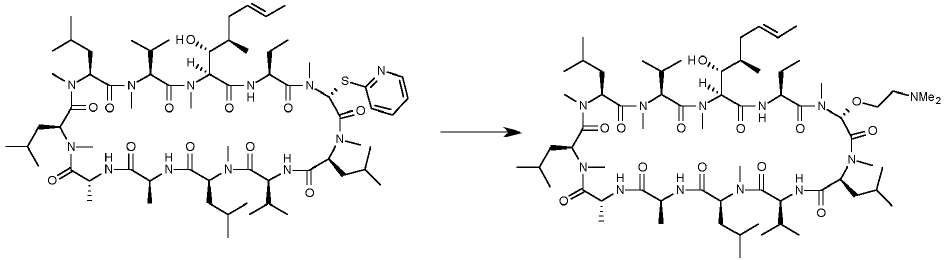

### [2'-(2-Dimethylaminoethoxy)-Sar]³-cyclosporine A (1)

Copper triflate (0.291 g, 0.8 mmol) and 3 angstrom molecular sieves were added to a flask, dry THF (3 mL) was added and the flask was flushed with argon. In a separate flask, a mixture of [2'-(2-thiopyridyl)-Sar]³-cyclosporine A (1a) (0.293 g, 0.223 mmol), dimethylaminoethanol (0.086 g, 0.96 mmol) and 3 A molecular sieves in dry THF (2 mL) was stirred for 30 minutes and then added to the copper triflate solution. The reaction was allowed to stir at room temperature overnight. A saturated solution of NaHCO₃ (10 mL) was added and the mixture was filtered through celite. The celite was washed with ethyl acetate (3 × 25 mL) and added to the filtrate. The organic layer was separated; the aqueous was extracted with EtOAc (2 × 25 mL) and the combined organic fractions were dried over anhydrous Na₂SO₄ and evaporated. Purification of the crude material on silica gel afforded the title compound, 86.4 mg. ¹H NMR (400 MHz, CHLOROFORM-d) d 7.92 (d, *J*=9.61 Hz, 1H), 7.75 (d, *J*=7.32 Hz, 1H), 7.22 (d, *J*=8.15 Hz), 7.15 (d, *J*=7.86 Hz,), 6.01 (s, 1H), 5.70 (dd, *J*=4.22, 10.86 Hz, 1H), 5.46 (d, *J*=6.10 Hz, 1H), 5.35 (q, *J*=4.77 Hz, 1H), 5.27 (dd, *J*=4.15, 11.42 Hz, 1H), 5.14 (d, *J*=10.83 Hz, 1H), 5.05-5.11 (m, 1H), 4.94-5.04 (m, 1H), 4.77-4.90 (m, 1H), 4.73 (s), 4.66 (t, *J*=8.83 Hz, 1H), 4.46-4.57 (m, 1H), 3.71-3.81 (m, 1H), 3.58-3.67 (m, *J*=5.15, 5.64, 5.64, 5.83 Hz, 1H), 3.53-3.58 (m, 1H), 3.51 (s, 2H), 3.24 (s, 2H), 3.20 (s, 2H), 3.13 (d, *J*=2.10 Hz, 3H), 2.71 (d, *J*=6.54 Hz, 3H), 2.49-2.67 (m, 2H), 2.33-2.46 (m, 1H), 2.27 (s, 4H), 1.88-2.20 (m, 4H), 1.74 (d, *J*=0.29 Hz, 6H), 1.57-1.68 (m, 5H), 1.38-1.52 (m, 2H), 1.35 (d, *J*=7.27 Hz, 3H), 1.26 (d, *J*=2.88 Hz, 4H), 0.77-1.12 (m, 30H), 0.70 (d, 2H).

The skilled person will for example appreciate that analogues of Compound 1 can be made by using different amino alcohol reagents. For example, the number of carbon atoms between the alcohol and amine group could be increased or decreased (examples of linking groups include, methylene, ethylene, propylene, butylene, pentalene, and may include branched versions thereof, such as iso-propylene, sec-butylene, tert-butylene, 2-methylbutylene, 2,2-dimethylpropylene). Alternatively, or in addition, the N-amino substituents on the amino alcohol could also be changed to give further analogues of Compound 1 (examples of N-amino substituents include, methyl, ethyl, propyl, isopropyl, n-butyl, sec-butyl, tert-butyl).

### Preparation of [2'-(2-N-Boc-aminoethoxy)-Sar]3-cyclosporine A

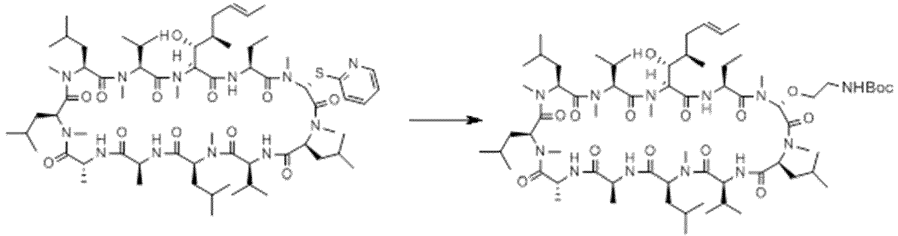

Copper triflate (4.95 g, 13.7 mmol) and 3 A molecular sieves were suspended in anhydrous THF (50 mL) and stirred under argon for 30 min. A solution of [2'-(2-thiopyridyl)-Sar]³-cyclosporine A (1) (5.0 g, 3.82 mmol) and N-Boc-ethanolamine (2.64 g, 16.4 mmol) in anhydrous THF (10 mL) was dried over 3 A molecular sieves for 30 min and then added to the copper triflate suspension. The resulting mixture was stirred at room temperature overnight. Saturated NaHCO₃ (2 × 50 mL) was added and the mixture was filtered through Celite. The Celite pad was washed with EtOAc (4 × 100 mL) and the organic layer was separated. The aqueous phase was extracted with EtOAc (2 × 50 mL) and the combined organic fractions were washed with saturated NaCl (50 mL), dried over anhydrous Na₂SO₄ and evaporated. The crude material was purified on silica to yield the title compound, 4.18 g. ¹H NMR (400 MHz, CHLOROFORM-d) d ppm 0.72 (ddd, 2 H) 0.91 (m, 31 H) 1.32 (m, 8 H) 1.48 (dddd, J=3.95, 3.07, 2.23, 0.95 Hz, 2 H) 1.69 (m, 10 H) 2.10 (m, 4 H) 2.39 (m, 1 H) 2.70 (m, 4 H) 2.95 (m, 2 H) 3.12 (d, J=7.42 Hz, 4 H) 3.17 (d, J=9.37 Hz, 1 H) 3.20 (s, 2 H) 3.25 (s, 2 H) 3.29 (m, J=6.69, 3.02, 1.45, 0.76, 0.63 Hz, 1 H) 3.41 (m, 1 H) 3.51 (s, 2 H) 3.61 (m, 1 H) 3.75 (dddd, J=7.73, 1.54, 1.02, 0.73 Hz, 1 H) 4.13 (q, J=7.11 Hz, 1 H) 4.50 (m, 1 H) 4.65 (dd, J=18.06, 0.44 Hz, 1 H) 4.98 (m, 4 H) 5.30 (m, 2 H) 5.47 (m, 1 H) 5.70 (m, 1 H) 5.93 (d, J=0.34 Hz) 7.21 (m, 1 H) 7.71 (m) 8.03 (m).

### Preparation of [2'-(2-aminoethoxy)-Sar]³-cyclosporine A

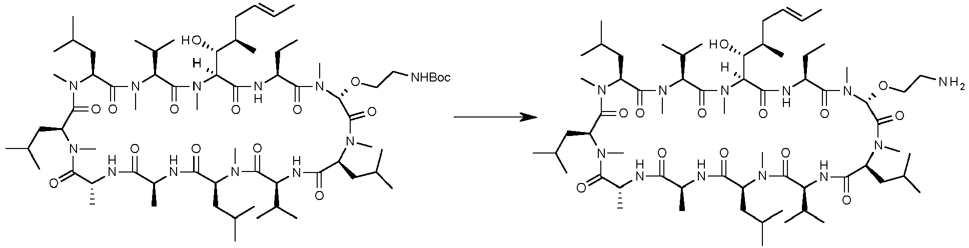

A solution of [2'-(2-N-Boc-aminoethoxy)-Sar]³-cyclosporine A (3) (3.0 g, 2.2 mmol) in dry CH₂Cl₂ (30 mL) was cooled to 0 C and trifluoroactetic acid (6.54 mL, 10.03 g, 88 mmol) was added dropwise and the mixture was stirred for 30 min. The solvent was evaporated and the crude material was purified on silica to deliver the title compound, 1.99 g.

### Preparation of [2'-(2-Dimethylaminoethoxy)-Sar]³-cyclosporine A (2)

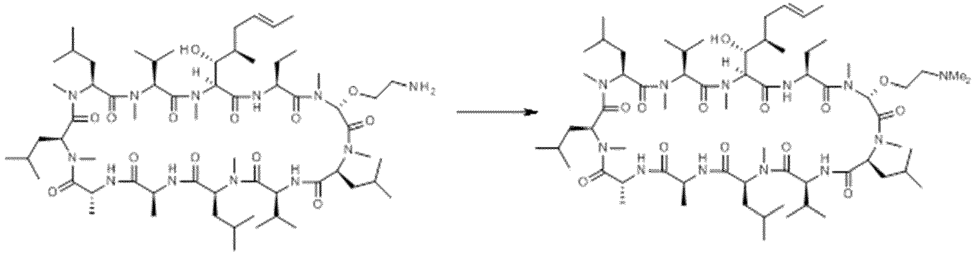

[2'-(2-Aminoethoxy)-Sar]³-cyclosporine A (0.273 g, 0.216 mmol) was dissolved in CH₂Cl₂ (5 mL) and formaldehyde (37% aqueous sol., 0.048 mL, 0.69 mmol) was added followed by NaB(OAc)₃H (0.138 g, 0.649 mmol) and the reaction was allowed to stir at about room temperature for 18h. The reaction mixture was filtered through a small pad of silica gel which was washed with 90:9:1 CH₂Cl_{2:}MeOH:conc.NH₄OH (5 × 100 mL). The solvent was evaporated and the product was isolated by chromatography on silica gel to afford the title compound, 0.214 g.

### Cyclophilin inhibition binding measurements

The cyclophilin inhibition binding of compounds disclosed herein was determined using a competitive ELISA adapted from the methods described by Quesniaux et al. (Eur. J Immunol., 1987, 17:1359-1365). Activated ester of succinyl spacers bound to D-Lys⁸-cylosporine A (D-Lys⁸-Cs) are coupled to bovine serum albumin (BSA) through D-lysyl residue in position 8. BSA is dissolved in 0.1 M borate buffer, pH 9.0 (4 mg in 1.4 ml). A hundredfold molar excess of D-Lys⁸-Cs dissolved in dimethyl formamide (0.6 ml) is added drop wise to the BSA under vigorous stirring. The coupling reaction is performed for 2 to 3 hours at room temperature under mild stirring and the conjugate is extensively dialyzed against phosphate-buffered saline (PBS, pH 7.4). After acetone precipitation of an aliquot of the conjugated protein, no covalently bound D-Lys⁸-Cs remains in the acetone solution and the extent of cyclosporine covalent binding is calculated.

Microtiter Plates are coated with D-Lys⁸-Cs-BSA conjugate (2 µg/ml in PBS for 24 hours at 4°C). Plates are washed with Tween^{®}/PBS and with PBS alone. To block nonspecific binding, 2% BSA/PBS (pH 7.4) is added to the wells and allowed to incubate for 2 hours at 37°C. A five-fold dilution series of the compound to be tested is made in ethanol in a separate microtiter plate. The starting concentration is 0.1 mg/mL for assays with human recombinant cyclophilin. 198 µL of 0.1 µg/mL cyclophilin solution is added to the microtiter immediately followed by 2 µL of diluted cyclosporine A (used as a reference compound) or the compound of the invention. The reaction between coated BSA-Cs conjugate, free cyclosporine A and cyclophilin is allowed to equilibrate overnight at 4°C. Cyclophilin is detected with anti-cyclophilin rabbit antiserum diluted in 1% BSA containing PBS and incubates overnight at 4°C. Plates are washed as described above. Bound rabbit antibodies are then detected by goat anti-rabbit IgG conjugated to alkaline phosphatase diluted in 1% BSA-PBS and allowed to incubate for 2 hours at 37°C. Plates are washed as described above. After incubation with 4-nitrophenyl phosphate (1 g/l in diethanolamine buffer, pH 9.8) for 1 to 2 hours at 37°C, the enzymatic reaction is measured spectrophotometrically at 405 nm using a spectrophotometer. The results are expressed as an EC₅₀, which is the concentration of the compound of the invention required to achieve 50% inhibition. Compound 1 had EC₅₀ values of less than 100nM against cyclophilins A, B and D.

### PPlase Inhibition

The assay was performed using an Agilent 8453 spectrophotometer essentially as described as the 'uncoupled assay' by Janowski *et al.* {Jankowski et al. Anal. Biochem. (1997), 252:299-307}. Assay buffer consisting of 35 mM HEPES pH 7.8 and 50 µM DTT was cooled to 10 °C (with stirring) in a precision glass cuvette and inhibitor was added from a 100% DMSO stock solution. A blank spectrum was obtained and then purified His tagged recombinant human cyclophilin enzyme (f/c 2 nM) and tetra peptide substrate, Suc-Ala-Ala-Pro-Phe-*para*nitroanilide dissolved in 0.5 M LiCI in trifluoroethanol (Bachem, f/c 60 µM) were added and the change in absorbance measured over 5 min at 330 nM. A first order rate equation was fitted to the absorbance data to obtain a rate constant (first 10 to 15 s were eliminated due to mixing). The catalytic rate was calculated from the enzymatic rate minus the background rate. The *K*ᵢ for an inhibitor was obtained from the rate constant plotted against the inhibitor concentration.

### Mitochondrial Permeability Transition

Mitochondrial Permeability Transition (MPT) is determined by measuring swelling of the mitochondria induced by Ca²⁺. The procedure is adapted from the method described by Blattner et al., 2001, Analytical Biochem, 295:220. Mitochondria are prepared from rat livers, which have been perfused with phosphate-buffered saline (PBS) to remove blood, using standard methods that utilize gentle homogenization in sucrose based buffer and then differential centrifugation to first remove cellular debris and then to pellet the mitochondria. Swelling is induced by 150 micro molar Ca²⁺ (added from a concentrated solution of CaCl₂) and is monitored by measuring the scattering at 535-540 nm. Representative compounds are added 5 minutes before swelling is induced. EC₅₀ is determined by comparing swelling with and without the compounds disclosed herein. Compound 1 inhibited mitochondrial swelling with an EC₅₀ of less than 0.2 uM.

### Acute Kidney injury

Compound 1 and Cyclosporin A formulations were prepared by mixing these compounds with Cremophor/saline/DMSO.

Sprague-Dawley rats were divided into six groups: Group (i) the sham group, dosed with Cremophor/saline/DMSO with no active; Group (ii) the control group, dosed with Cremophor/saline/DMSO with no active; Group (iii) dosed with Compound 1 (3 mg/kg); Group (iv) dosed with CsA (3 mg/kg); Group (v) dosed with Compound 1 (10 mg/kg); Group (vi) dosed with CsA (10 mg/kg). With the exception of Group (i), i.e. the 'sham group', renal Ischemia-Reperfusion Induced Acute Kidney Injury (AKI) was induced in the rats by ligation of bilateral renal arteries for 30 min and then ligation was released.

Animals in the control and treatment groups were administered intraperitoneal injections three times (1h before ligation, 4h and 8h after ligation). Blood was taken from the animals 24 hours after the ligation/release procedure and analyzed for serum **Creatinine** and **Blood Urea Nitrogen** (BUN) concentrations, as a measure of kidney injury.

The results of those experiments are shown below, and graphically in Figures 1 and 2.

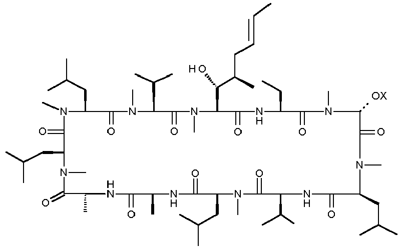

**Table 1 - Measurement of Cyclophilin A inhibition, Cyclophilin D inhibition and Mitochondrial Permeability Transition (MPT).**

| **Entry** | **X** | **CypA EC₅₀ (nM)** | **CypD EC₅₀ (nM)** | **MPT (µM)** |
|---|---|---|---|---|
| **1** | 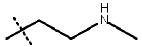 | 61 | 2170 | 10 |
| **2** | 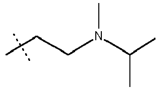 | 202 | 3550 | 7.6 |
| **3** | 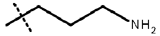 | 14 | ND | 2.69 |
| **4** | 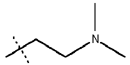 | 60 | 24 | 0.1 |
| *Compound 1* | | | | |
| **5** | 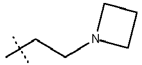 | 66 | ND | 10 |
| **6** | 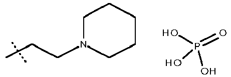 | 118 | 2500 | 7.5 |
| **7** | 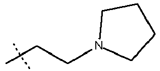 | 12 | 1200 | 10 |

The results displayed in Table 1 demonstrate the unexpectedly high Cyclophilin D inhibition and MPT of Compound 1 (entry 4) relative to similar analogues (entries 1-3 and 5-7). A 100 fold improvement in MPT was observed relative to three other compounds (entries 1, 5 and 7) and an over 25 fold improvement was observed relative to the next best performing analogue (entry 3). Compound 1 also displayed superior Cyclophilin D inhibition, with at least a 50 fold improvement relative to all other analogues tested.

**Table 2 - Measurement of serum Creatinine and BUN concentrations of Groups (i) to (vi)**

| | **Creatinine (umol/L)** | **Blood Urea Nitrogen (mmol/L)** |
|---|---|---|
| Group (i) | 25 | 5 |
| Group (ii) | 195 | 38 |
| Group (iii) | 60 | 14 |
| Group (iv) | 115 | 22 |
| Group (v) | 250 | 40 |
| Group (vi) | 290 | 42 |

LPS induced Acute Kidney Injury (AKI) was induced in mice (C57) by intraperitoneal injection of LPS (15 mg/kg). Twenty mice were randomly divided into two groups. Animals in the control group received vehicle (Cremophor/saline/DMSO) and the treatment group received Compound 1 (3 mg/kg in Cremophor/saline/DMSO) each dosed intraperitoneally. The animals were dosed with vehicle or Compound 1 three times (1 h before LPS injection and 4h and 8h after LPS injection) and blood was taken from the animals 12 h after LPS injection. The activity of the compound was determined by increased survival rate and by evaluation of markers of kidney function.

### Discussion of results

In Figure 1 the blood serum Creatinine concentration is indicative of kidney damage. The 'sham group' are rats without induced AKI. The 'control group' represents rats with induced AKI, and which are untreated. Therefore, it can be seen that induced AKI results in increased levels of Creatinine from 25 umol/ml (Group 1) to 195 umol/ml (Group 2).

Treating rats with induced AKI with 3 mg/kg of CsA (Group 4) results in the Creatinine levels dropping from 195 umol/ml to 115 umol/ml as compared to Group 2. Therefore, it is understood that CsA is acting to prevent the ischaemia-reperfusion injury.

Surprisingly, when rats with induced AKI are treated with 3 mg/kg of Compound 1 (Group 3), this gives a very marked reduction in Creatinine levels, dropping from 195 umol/ml to 60 umol/ml (as compared to Group 2), which is approaching the Creatinine levels seen in the 'sham group' (Group 1), i.e. rats with no induced AKI. When the dose of Compound 1 and CsA are increased from 3 mg/ml (Groups 3 and 4) to 10 mg/ml (Groups 5 and 6), it appears that the benefit of the CsA and Compound 1 are reduced, with Compound 1 still performing better than CsA.

In Figure 2 the Blood Urea Nitrogen (BUN) concentrations is indicative of kidney damage.

Figure 2 follows the same trend as seen in Figure 1. That is, 3 mg/kg of CsA results in a drop in BUN levels (Group 4 compared to Group 2), whereas 3 mg/kg of Compound 1 shows a very marked reduction in BUN levels (Group 3 compared to Group 2), getting towards the BUN level seen in the 'sham group' (Group 1). Increasing the concentration of Compound 1 and CsA from 3 mg/kg (Groups 3 and 4) to 10 mg/kg (Groups 5 and 6) proves to be less effective. This result supports the result seen in Figure 1.

### Necrotic cell death of murine pancreatic acinar cells by propidium iodide

In order to assess activation of necrotic cell death pathway, 1 µM propidium iodide (PI; λex: 543 nm, λem: 610-690 nm) was used to evaluate plasma membrane rupture. In order to evaluate the inhibition of necrosis by a potential drug, freshly isolated pancreatic acinar cells (PAC) from a CD1 mouse were divided into the four following groups:
1. Control group; PACs incubated with sodium HEPES and vehicle (0.5% DMSO).
2. Taurolithocholate acid sulphate (TLCS) group; PACs incubated with 500 µM TLCS and vehicle.
3. Potential drug only group (for cytotoxicity test); PACs incubated with the potential drug.
4. Potential drug + TLCS group; PACs incubated with the potential drug in the presence of 500 µM TLCS.

For each group, PACs were incubated for 30 minutes at room temperature with gentle shaking. For each group, 8 randomly selected fields (each field contained mostly over 100 cells) of view under confocal microscope were taken of each mouse isolate, and the total number of cells displaying PI uptake were counted per field to give a percentage ratio for each field, averaged across fields, and converted to a mean and standard error of mean for a minimum of three mice per experimental group. The whole assay was performed in a blinded fashion in such a way that the observer choosing the fields and the observer undertaking image analysis were unaware of the treatment groups.

### In Vivo Mouse TLCS Acute Pancreatitis Model

The experiment measures the ability of a compound to rescue bile acid, taurolithocholylsulphate (TLCS) induced acute pancreatitis in a mouse model. *In vivo* animal protocols are approved by the UK Home Office. C57BL6/J mice (Charles River UK Ltd) are acclimated for at least 1 week before *in vivo* experiments. Compounds are made up in the appropriate formulation. TLCS-AP is induced by retrograde pancreatic duct injection of 3 mM TLCS as described previously [Laukkarinen *et al.,* 2007]. Compound is typically administered by i.p. injection, and then animals sacrificed 24h later. Histology was visualised and serum amylase, interleukin 6 and myeloperoxidase activity were measured as described previously [Mukherjee et al, 2016].

Serum amylase levels were determined using a Roche automated clinical chemistry analyzer (Roche).

Serum IL-6 levels were determined by Quantikine mouse ELISA kit (R&D systems).

Myeloperoxidase (MPO) activity was used as a marker of neutrophil infiltration and determined as described. Pancreatic and lung tissue were first homogenized and resuspended in 100 mM potassium phosphate buffer (pH 7.4) containing protease inhibitor, after repeating centrifugation and resuspension 2-3 times, then further resuspended in 100 mM potassium phosphate buffer (pH 5.4) containing 0.5% hexadecyltrimethyl ammonium bromide, 10 mM EDTA and protease inhibitors, then freeze-thawed three times, sonicated for 30 sec and finally centrifuged for 15 min at 16,000 × g. The supernatant was collected and MPO activity with general peroxidase substrates 3,3,5,5-tetramethylbenzidine was measured using H₂O₂. Absorbance was measured at 655 nm. The homogenate protein level was detected using BCA assay kit. MPO activity was normalized based on the protein level of each sample and mean value of TLCS group.

### Histology

For morphological examination, pancreatic tissues were fixed in 10% formalin, embedded in paraffin, and stained with hematoxylin and eosin (H&E). Histopathological evaluation was assessed blindly on 10 random fields (×10 high power fields) of each slide by two independent investigators, grading the degree and extent of oedema, inflammatory infiltration and necrosis from 0 to 3 [5], calculating summated mean ± s.e.m.

### Discussion of results

In summary, Compound 1 was found to be surprisingly efficacious in the treatment or prevention of pancreatitis when compared to similar compounds.

In overall summary, Compound 1 was found to be surprisingly efficacious in the treatment or prevention of ischaemia-reperfusion injury, in particular at lower concentration levels. The compound is also particularly efficacious against acute kidney injury and pancreatitis.

## Claims

1. A compound for use in the treatment or prevention of acute or chronic inflammatory disorders wherein the compound is Compound 1: 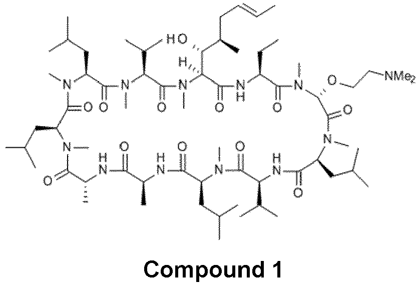
or a salt thereof;
and wherein the disorder is selected from acute kidney injury, chronic or acute pancreatitis and ischaemia-reperfusion injury wherein the ischaemia-reperfusion injury occurs after reattachment of a severed body part or during the process of organ transplantation.

2. The compound for use according to claim 1 wherein the disorder is acute kidney injury.

3. The compound for use according to claim 1 wherein the disorder is ischaemia-reperfusion injury which occurs after re-attachment of a severed body part or during the process of organ transplantation.

4. The compound for use according to claim 1 wherein the disorder is chronic or acute pancreatitis.

5. The compound for use according to any one of claims 1 to 4, wherein the dose of the compound is 0.1 to 10 mg/kg.

6. The compound for use according to claim 5, wherein the dose of the compound is 1 to 3 mg/kg.

7. A method of preparing Compound as defined in claim 1, the method comprising a product forming reaction, the reaction comprising copper triflate and N,N-dimethylaminoethanol.

8. The method of claim 7, wherein the reaction comprises a drying agent and/or is performed under substantially anhydrous conditions; and wherein optionally the drying agent is molecular sieves.

9. The method of claim 7 or 8, wherein the N,N dimethylaminoethanol reacts with a cyclosporine precursor compound comprising a labile group, wherein the labile group is lost in the reaction; wherein optionally the labile group is bonded to the precursor compound by a -S- bond; and further optionally the labile group is a thiopyridyl group or a mercaptobenzthiazole-2-ylthio group.

## Patentansprüche

1. Verbindung zur Verwendung bei der Behandlung oder Vorbeugung von akuten oder chronischen entzündlichen Erkrankungen, wobei die Verbindung Verbindung 1 ist: 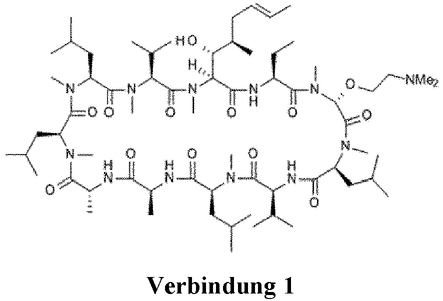
oder Salz davon;
und wobei die Erkrankung aus akuter Nierenschädigung, chronischer oder akuter Pankreatitis und Ischämie-Reperfusionsschaden ausgewählt ist, wobei der Ischämie-Reperfusionsschaden nach Wiederverbinden eines abgetrennten Körperteils oder während des Prozesses einer Organtransplantation auftritt.

2. Verbindung zur Verwendung nach Anspruch 1, wobei die Erkrankung eine akute Nierenschädigung ist.

3. Verbindung zur Verwendung nach Anspruch 1, wobei die Erkrankung ein Ischämie-Reperfusionsschaden ist, der nach Wiederverbinden eines abgetrennten Körperteils oder während des Prozesses einer Organtransplantation auftritt.

4. Verbindung zur Verwendung nach Anspruch 1, wobei die Erkrankung chronische oder akute Pankreatitis ist.

5. Verbindung zur Verwendung nach einem der Ansprüche 1 bis 4, wobei die Dosis der Verbindung 0,1 bis 10 mg/kg beträgt.

6. Verbindung zur Verwendung nach Anspruch 5, wobei die Dosis der Verbindung 1 bis 3 mg/kg beträgt.

7. Verfahren zum Herstellen einer Verbindung nach Anspruch 1, wobei das Verfahren eine Produktbildungsreaktion umfasst, wobei die Reaktion Kupfertriflat und N,N-Dimethylaminoethanol umfasst.

8. Verfahren nach Anspruch 7, wobei die Reaktion ein Trocknungsmittel umfasst und/oder unter im Wesentlichen wasserfreien Bedingungen durchgeführt wird; und wobei es sich bei dem Trocknungsmittel optional um Molekularsiebe handelt.

9. Verfahren nach Anspruch 7 oder 8, wobei das N,N-Dimethylaminoethanol mit einer Cyclosporin-Vorläuferverbindung reagiert, die eine labile Gruppe umfasst, wobei die labile Gruppe in der Reaktion verloren geht; wobei optional die labile Gruppe durch eine -S-Bindung an die Vorläuferverbindung gebunden ist, und ferner optional die labile Gruppe eine Thiopyridylgruppe oder eine Mercaptobenzthiazol-2-ylthiogruppe ist.

## Revendications

1. Composé pour utilisation dans le traitement ou la prévention de troubles inflammatoires aigus ou chroniques, où le composé est le Composé 1 : 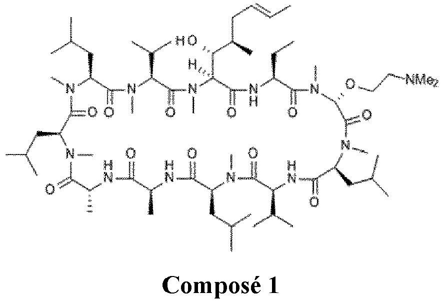
ou un sel de celui-ci ;
et où le trouble est sélectionné parmi : lésion rénale aiguë, pancréatite chronique ou aiguë et lésion de reperfusion faisant suite à une ischémie, où la lésion de reperfusion faisant suite à une ischémie se produit après le regreffage d'une partie du corps sectionnée ou au cours d'une greffe d'organe.

2. Composé pour utilisation selon la revendication 1, où le trouble est une lésion rénale aiguë.

3. Composé pour utilisation selon la revendication 1, où le trouble est une lésion de reperfusion faisant suite à une ischémie, qui se produit après le regreffage d'une partie du corps sectionnée ou au cours d'une greffe d'organe.

4. Composé pour utilisation selon la revendication 1, où le trouble est une pancréatique chronique ou aiguë.

5. Composé pour utilisation selon l'une quelconque des revendications 1 à 4, où la dose du composé est de 0,1 à 10 mg/kg.

6. Composé pour utilisation selon la revendication 5, où la dose du composé est de 1 à 3 mg/kg.

7. Procédé de préparation d'un composé tel que défini dans la revendication 1, le procédé comprenant une réaction formant le produit, la réaction comprenant le triflate de cuivre et le N,N-diméthylaminoéthanol.

8. Procédé selon la revendication 7, où la réaction comprend un desséchant et/ou est réalisée dans des conditions essentiellement anhydres ; et où optionnellement le desséchant est constitué de tamis moléculaires.

9. Procédé selon la revendication 7 ou 8, où le N,N diméthylaminoéthanol réagit avec un composé précurseur de la cyclosporine comprenant un groupe labile, où le groupe labile est perdu dans la réaction ; où optionnellement le groupe labile est lié au composé précurseur par une liaison -S- ; et, en outre, où optionnellement le groupe labile est un groupe thiopyridyle ou un groupe mercaptobenzthiazole-2-ylthio.
